# EUROPEAN PATENT SPECIFICATION

(11) **EP 3 285 727 B1**
(45) Date of publication and mention of the grant of the patent: **18.12.2019**
(21) Application number: 16719723.5
(22) Date of filing: 22.04.2016
(51) Int. Cl.: A61K 8/34, A61K 8/44, A61K 8/46, A61Q 13/00, A61Q 19/10, A61K 8/06, A61Q 5/02

(54) **CONCENTRATED PERSONAL CLEANSING COMPOSITIONS AND METHODS**
KONZENTRIERTE KÖRPERPFLEGEZUSAMMENSETZUNGEN UND VERFAHREN
COMPOSITIONS D'HYGIÈNE PERSONNELLE CONCENTRÉES ET MÉTHODES ASSOCIÉES

(30) Priority: 23.04.2015 US 201562151900 P
(43) Date of publication of application: 28.02.2018
(73) Proprietor: The Procter & Gamble Company, Cincinnati, OH 45202 (US)
(72) Inventor: SMITH, Edward, Dewey, III, Cincinnati, Ohio 45202 (US); MCCONAUGHY, Shawn, David, Cincinnati, Ohio 45202 (US); LI, Jianjun, Justin, Cincinnati, Ohio 45202 (US); FLICKINGER, Marc, Adam, Cincinnati, Ohio 45202 (US)
(74) Representative: P&G Patent Germany
(86) International application number: PCT/US2016/028840
(87) International publication number: WO 2016/172478

(56) References cited:
- WO-A1-2014/090959
- FR-A1- 2 998 476
- US-A- 5 374 614

## Description

### FIELD OF THE INVENTION

This application relates to rinse-off cleansing compositions with surfactant, perfume, solvent, and water; and methods relating thereto.

### BACKGROUND OF THE INVENTION

Cleansing is an activity that has been done for thousands of years. Early cleansers were based on either soap chemistry or simple mechanical action in order to remove dirt from the skin, as well as endogenous soils such as sweat, sebum and body odors. Smelling clean is an important benefit but early cleansers did not provide perfume to the skin during cleansing as it would have been wasteful for a very expensive ingredient, the perfume. Instead, perfume was applied after cleansing. As skin cleansing compositions have become more complex, providing scent during cleansing and residual scent on the skin after cleansing are expected by users of modern skin cleansers. U.S. Patent 5,374,624 relates to clear o/w microemulsions comprising a perfume oil, an aqueous phase and one or more surfactants with HLB between 9 and 18, and co-surf actants of which at least 0.5% of ionic co-surfactant. As such, improved cleansing compositions which can provide scent during cleansing and/or residual scent on the skin are desired.

### SUMMARY OF THE INVENTION

Use of a hydric solvent to enhance fragrance longevity on skin of a rinse-off cleansing composition, wherein the composition comprises: a) from 35% to 85%, by weight of the composition, of surfactant, wherein the surfactant comprises a first surfactant and a cosurfactant; b) from 4% to 30%, by weight of the composition, of a perfume, wherein the weight percent of perfume is from 8% to 90%, by weight of the surfactant; c) from 6% to 20%, by weight of the composition, of a hydric solvent, wherein the hydric solvent comprises dipropylene glycol, diethylene glycol, dibutylene glycol, hexylene glycol, butylene glycol, pentanediol, heptanediol, propylene glycol, a polyethylene glycol having an average molecular weight below 500, or a combination thereof, and wherein the weight percent of the hydric solvent is from 16% to 24%, by weight of the surfactant; and d) from 2% to 57%, by weight of the composition, of water; wherein the rinse-off cleansing composition is not a ringing gel, and wherein the composition is a microemulsion or contains a microemulsion phase.

A method of enhancing fragrance longevity on skin of a rinse-off cleansing composition, comprising, combining: a) from 35% to 85%, by weight of the composition, of surfactant; b) from 4% to 30%, by weight of the composition, of a perfume, wherein the weight percent of perfume is from 12% to 40%, by weight of the surfactant; c) from 6% to 20%, by weight of the composition, of a hydric solvent, wherein the hydric solvent comprises dipropylene glycol, diethylene glycol, dibutylene glycol, hexylene glycol, butylene glycol, pentanediol, heptanediol, propylene glycol, a polyethylene glycol having an average molecular weight below 500, or a combination thereof, preferably dipropylene glycol, and wherein the weight percent of the hydric solvent is from 16% to 24%, by weight of the surfactant; and d) from 2% to 57%, by weight of the composition, of water; to form the cleansing composition; wherein the rinse-off cleansing composition is not a ringing gel.

These and other methods and compositions will be more fully understood in light of the detailed description below.

### BRIEF DESCRIPTION OF THE DRAWINGS

FIG. 1 is a graph of total GCMS peak area versus the dilution ratio of water to composition measured in accordance with the Perfumed Headspace Abundance During Dilution Method; and
FIG. 2 is a graph showing the equilibrium solubility of a particular perfume at room temperature by % T at 640 nm to moles perfume/moles surfactant.

### DETAILED DESCRIPTION OF THE INVENTION

### I. Definitions

As used herein, the following terms shall have the meaning specified thereafter:
"Cleansing composition" refers to compositions intended for topical application to the skin or hair for cleansing.
"Concentrate/concentrated" as used herein with respect to a cleansing composition refers to a composition where the weight percentage of surfactant relative to the total composition is greater than 15%.
"Free of' refers to no detectable amount of the stated ingredient or thing.
"Gel" refers to a material or composition that does not flow under its own weight and has a G' greater than 25 Pa at 10 Hz in an oscillatory rheology test.
"Hydric solvent" refers to a solvent that is neutral organic species that contains at least 2 hydroxyl groups and is not a hydrotrope.
"Hydrotrope" refers to a charged, amphiphilic solubility modifier. Hydrotropes are generally charged olefins especially an olefin sulfonate such as an aromatic sulfonate.
"In-vitro bloom" refers to the amount of perfume experienced at a 3:1 by weight water to composition dilution versus the amount of perfume in the headspace prior to dilution and can be measured in accordance with Perfumed Headspace Abundance During Dilution Method set out below.
"Micelle" as used herein refers to a structure comprising individual surfactant molecules aggregated to form a hydrophobic core region with externally facing polar head groups in equilibrium with surfactant monomers in a polar phase, having a characteristic dimension that is a single digit multiple of the surfactant length, i.e., generally less than 10 nm in diameter.
"Microemulsion" as used herein refers to a thermodynamically stable isotropic mixture of oil, surfactant, and water comprising an interior hydrophobic core, having a size greater than 10 nm diameter.
"Perfume" refers to a mixture of volatile organic oils having a pleasant aroma wherein the perfume components have individual molecular weights between 75 and 400 Daltons.
"Relative Bloom" refers to perfume in the headspace over a composition during use for a perfumed cleansing composition relative to concentration for a conventional, hydric solvent free, control micelle composition having 10 wt% starting surfactant and 1 wt% starting perfume when the same perfume is used in the composition and the micelle composition.
"Rinse-off' means the intended product usage includes application to skin and/or hair followed by rinsing and/or wiping the product from the skin and/or hair within a few seconds to minutes of the application step. The product is generally applied and rinsed in the same usage event, for example, a shower.
"Room temperature" refers to a temperature of 25°C.
"Solvent" refers to species or mixture of species present in a molecular solution in the greatest molar concentration acting in a way to dissolve other species, the latter species generally being larger molecules. A "hydric solvent" is a water miscible solvent.
"Single Phase" when used herein with respect to inventive cleansing compositions refers to homogeneity when measured at the designated temperature in accordance with the Ultracentrifuge Test.
"Stable" when used herein with respect to the inventive cleansing compositions refers to visual stability when measured in accordance with the Stability Test herein.
"Substantially free of" refers to 2% or less, 1% or less, or 0.1% or less of a stated ingredient.
"Surfactant" as used herein refers to amphiphilic molecules which can aggregate to form micelles and other surfactant structures, which are soluble in an aqueous phase and contribute to foaming during a cleansing event, i.e., stabilizing an air interface.

### II. Cleansing Compositions

Modern consumers of cleansing compositions expect the composition to provide scent both during use and to have residual scent on the skin after use, making perfume an important component of cleansing compositions. Perfume is also an important component of many skin cleansers to mask the base odor of cleansing ingredients, which can be unpleasant.

Perfume is hydrophobic, whereas skin cleansers generally have an aqueous, continuous phase which provides essentially no ability to carry perfume. It is desirable to provide perfume in a soluble form in a liquid skin cleanser, since insoluble phases of any kind can lead to instability problems in the composition. Perfume is therefore generally solubilized within the surfactant component of cleansers, such as micelles, lamellar structures, vesicles and the like. Surfactant structures of all kinds contain hydrophobic regions due to the aggregation of surfactant tails, which are able to solubilize significant quantities of perfume oil. Perfume generally exists within the surfactant tails as a molecular solution due to the interaction of the perfume with the surfactant tails, not as a colloidal structure such as an emulsion droplet, which is not thermodynamically stable.

A problem exists in providing perfume scent during use and residual scent to the skin from skin cleansers. Well known physical laws govern the relationship between perfume in the air in equilibrium with perfume solubilized in a micelle or other environment. This relationship is defined by the mole fraction of perfume in the soluble environment, generally the micelle. Micelles are common features of skin cleansers since even non-micellar surfactant generally become micelles during the dilution experienced while cleansing.

Since the perfume concentration in a skin cleanser is generally only 25% or less on a molar basis in the surfactant micelle, the vapor pressure of each perfume molecule can be reduced by 75% or even more, due to its solubilization in the micelle. The desire to deliver perfume to the skin suffers from a similar fate during cleansing. Perfume molecules can diffuse, or partition into the skin during cleansing. The driving force to do so is the thermodynamic activity coefficient gradient for the perfume molecules. While a pure perfume applied to the skin, having an activity coefficient of 1, can partition quickly into skin, perfume located in a surfactant micelle proximal to the skin suffers from the activity coefficient reduction (75% or more) due to micellar solubilization. Therefore most perfume in cleansing compositions (between 50-90%) generally is washed away during rinsing before a significant amount can partition into the skin or bloom into the headspace. The result is the skin retains no or very little scent and only for a short duration after a typical cleansing event. Thus, delivery of perfume to the air and to the skin during cleansing is inefficient and therefore expensive.

Overcoming these technical constraints in order to increase initial perfume perception in a neat composition, perfume delivery to the air during cleansing, and to the skin is not simply a matter of adjusting formula components at increased cost. Natural limits exist related to factors such as solubility. For example, increasing perfume in a composition is not only impractical from a cost standpoint, perfume being quite expensive, but is also infeasible considering the abundance of perfume can quickly become insoluble in the surfactant composition, leading to instability. Formulating a perfume to contain more high vapor pressure components to enhance bloom may be useful, but does not overcome the innate vapor pressure reduction of perfume due to solubilization in the micelle, in addition to other restrictions to the scent character that would result. Benefits of these approaches are limited.

Various means to overcome this problem have been suggested. Perfume microcapsules have been developed to encapsulate perfume and protect it from contact with surfactant. However, only a limited number of perfume molecules are stable in perfume microcapsules; and the perfume microcapsule itself must then be delivered to the skin and, later, mechanically crushed by the consumer in order to release the perfume. Most perfume microcapsules are themselves washed down the drain during cleansing, affording little benefit.

Additionally, cleansing compositions have been formulated as micelles. Surfactants have a critical micelle concentration, or CMC, at which they aggregate. Below the CMC surfactant exists as monomers in solution. It has been suggested that dilution to below the CMC can release perfume to increase bloom. The problem with this approach is the CMC is very low, often 100 ppm for cleansing surfactant mixtures (i.e., 0.01 wt.%, a dilution of more than 500-fold from an original composition). Thus, the CMC occurs at concentrations not relevant to cleansing nor rinsing the body. During rinsing, the CMC is reached only at the very end of cleansing, by which time nearly all the cleansing components have already been washed down the drain in the form of micelles, carrying the perfume with them. Relevant dilutions during cleansing are less than 10-fold, especially less than 5-fold, during which time there is extensive exposure of the wash composition to the body and to the air in the shower, affording both time and opportunity for perfume to bloom and partition to the skin, if it can be removed from the environment of the micelle.

A constraint in overcoming these problems relates to the rheology profile of the composition. Liquid skin cleansing compositions generally utilize dosing from a package onto a hand, a cleansing implement, or the skin itself without running off. Further, compositions should spread easily across the body to effect thorough cleansing. Preferred means to provide an acceptable rheology involve the use of surfactants to form elongated micelles or lamellar structures without the use of other rheology control agents, which can be wasteful and costly. Control of rheology using surfactants can provide important restrictions which limit the ability to modify surfactant mixtures to provide other benefits, such as perfume benefits. Often, polymeric or associative thickening agents can be used to control rheology, but these can create new restrictions or constraints by interacting with micelles. Overcoming the problems related to perfume benefits in cleansing compositions while maintaining an acceptable rheology profile for dispensing is therefore a highly constrained problem and difficult to overcome.

Surprisingly, inventors have discovered skin cleansing compositions can deliver enhanced initial perfume perception, perfume bloom during cleansing, and perfume retention on the skin for many hours after cleansing. Without wishing to be limited by theory, the enhanced perfume benefits are believed to result at least in part when at least a portion of the perfume in a composition exists in the physical form of a perfume microemulsion. In some cases, the microemulsion may be in equilibrium with other phases such as micelles or a lamellar phase. In some cases, the microemulsion can spontaneously form upon addition of water, i.e., during cleansing or rinsing. In the microemulsion form, it is believed most perfume is in a central core region and is not proximal to surfactant hydrocarbon, therefore it is not in a solvent-solute relationship which can reduce perfume activity coefficient. The result is bloom and/or relative bloom is significantly enhanced, sometimes doubled or even tripled or more; and scent of perfume over the skin after wash, can be enhanced by a similar magnitude.

To make a perfume microemulsion, sufficiency of perfume, which is the oil component for making a perfume core; the right level and mixture of surfactant; and a hydric solvent able to interact in a manner to make the microemulsion are believed to be contributing factors. The hydric solvent has multiple effects like, reducing the dielectric of the water phase, acting as a solvent for the surfactant head groups, reducing interfacial tension between the aqueous phase and hydrocarbon, and interacting with the perfume in the core. Hydric solvents can be chameleonic in nature, able to provide miscibility with hydrophobic perfume oil and water external to the microemulsion phase. During use of the body cleansing composition, as the composition is diluted, hydric solvent can be reduced in concentration in a perfume microemulsion core because of the abundance of water added during washing and rinsing, providing a further benefit to increase perfume activity coefficient by increasing perfume molar concentration in the core. Thus, a sufficient amount of particular kinds of hydric solvents can be used to form a microemulsion phase which can increase perfume activity during use.

Some compositions may be in a lamellar phase prior to dilution during use, but can be transformed to a perfume microemulsion during use. In some cases, the transformation during dilution to a perfume microemulsion may be brief, within a restricted dilution range, but such range is sufficient to deliver perfume bloom and partitioning into the skin which, once partitioning is effected, it cannot be reversed. Certain microemulsions may be in equilibrium with other phases, such as micelles or a lamellar phase, either in the composition or during dilution of the composition during use. There may be advantages for both the microemulsion and micelle phases to coexist, since micelles may provide superior lather and cleaning properties at the same time the microemulsion may deliver enhanced perfume benefits. Certain analytical measures, such as dynamic light scattering and optical light transmission, can be used as guides, when evaluating microemulsion phases. Additionally, perfume analysis in the headspace is directly relatable to the perfume solvent environment in a composition or a diluted composition, so that gas chromatography - mass spectrometry (GCMS) headspace measures are an indicator of the perfume environment, i.e., the microemulsion phase and the perfume relationship to solvent molecules therein. Well established physical laws govern the relationship between concentration of molecules in the headspace, and the solvent environment of the molecules in solution, e.g., Raoult's Law. Likewise, headspace measurements over the skin after washing are similarly useful, since perfume partitioning into the skin is enhanced by perfume activity coefficient, as previously discussed.

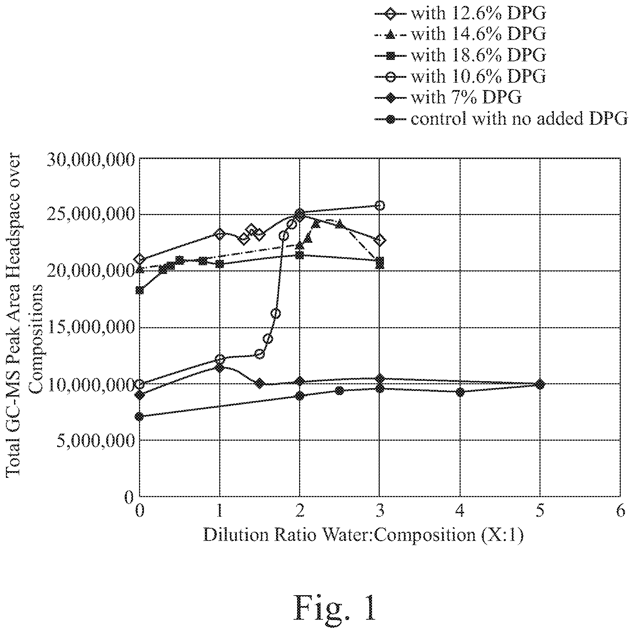

When the microemulsion phase is present, increased bloom and/or relative bloom can be demonstrated by measuring the relative abundance of perfume in the headspace over the composition or comparative composition using the Perfume Headspace Abundance During Dilution Method (PHADD), which utilizes solid phase microextraction GCMS (SPME-GCMS) to collect and evaluate perfume molecules (PRM) in the headspace over a neat composition and through stepwise aqueous dilutions. Results can be compared to a control micelle composition using the same perfume. When inventive compositions form a microemulsion phase, headspace perfume concentration can more than double compared to control, sometimes providing a 3-fold or greater headspace perfume concentration compared to control. See, for example, FIG. 1 which shows an enhancement of relative bloom (dilution of 0 on the X-axis) of a control micellar composition versus five inventive compositions with varying levels of hydric solvent (dipropylene glycol). FIG. 1 also shows an enhanced bloom (dilution of 3 on the X-axis) of the inventive compositions versus the control. In addition, evaluation among untrained and undirected consumers confirms extraordinary noticeability of the bloom benefit.

Increased perfume retention on the skin after cleansing can be demonstrated by measuring the relative abundance of perfume in the headspace over the skin using the Perfumed Skin Headspace Abundance Method (PSHAM), which utilizes a collector in a glass dome-shaped chamber over the skin to collect perfume, followed by heat desorption and evaluation of PRM by GCMS. Relative to control micelle compositions at the same perfume dose, inventive compositions can provide more than double the scent over the skin compared to control, sometimes providing a 3-fold or greater perfume retention on skin compared to control. Evaluation among untrained and undirected consumers confirmed extraordinary noticeability of the skin retention benefit.

Dynamic Light Scattering is a useful means to detect structures in the size range of microemulsion droplets, and micelles, but the results can be difficult to interpret when more than one structure may be present, such as micelles and a microemulsion both. A bimodal scattering intensity distribution may be present in inventive compositions, suggesting micelles having a diameter generally below 10 nm in equilibrium with larger structures, which are generally greater than 20 nm which are the perfume microemulsion droplets.

A microemulsion phase generally has a low viscosity and is a Newtonian fluid. Cleansing fluids with these viscosity characteristics are generally useful when dispensed from a package which controls the dose or spreads it onto the target surface, such as a pump foamer or a spray. To fit with current consumer habits during body cleansing, a cleansing composition can be in the form of a gel having a structure defined by an elastic modulus, G', a viscous modulus, G", a viscosity, and a shear thinning viscosity ratio as measured by the test methods below. A microemulsion composition can be concentrated to create a gel, wherein the gel provides a suitable rheology to easily dispense the composition from a package. The gel may comprise a lamellar phase, and may or may not have a high concentration of perfume in its headspace prior to being diluted, relative to a micelle composition. In some cases, when the gel has a high perfume concentration in its headspace, it is believed to be in equilibrium with a microemulsion phase, since the gel can evince a characteristic lamellar x-ray diffraction pattern. In other cases, the gel can have a high perfume concentration in the headspace only after dilution water is introduced.

The composition may be concentrated in order to create desirable rheology characteristics, i.e., a gel. Some compositions may be concentrated at least 3-fold relative to conventional body wash, which generally has 10 wt% surfactant. When the amount of surfactant is greater than 15 wt% of the composition, the surfactant can be considered to be concentrated and the composition can be considered to be a concentrate. Generally, a composition having 20 wt% surfactant can be considered a 2-fold concentrate, a composition having 30 wt% surfactant a 3-fold concentrate, and so on.

In certain cases, increasing concentration may be preferable because a gel can be created which has both desirable rheology characteristics useful for dispensing, in addition to a sufficiency of hydric solvent to form the microemulsion phase either as a component of the gel or during dilution of the composition. Some hydric solvents can significantly reduce the viscosity and/or G' of the gel, therefore concentration can be a useful means to increase the amount of hydric solvent that can be tolerated within a composition.

Additionally, organic solvents are useful to help form a microemulsion phase. Some organic solvents are miscible in water, at least partially miscible in perfume oil, and can interact with surfactant polar head groups to reduce structure and generally reduce viscosity as a result. The microemulsion phase requires very low interfacial tension. Perfume can be essentially relegated to the core of a water continuous microemulsion phase and therefore has a high activity coefficient, which can be effected by using a hydric solvent having water, perfume oil, and surfactant miscibility to reduce interfacial tension.

Water miscibility of hydric solvent can be determined by mixing the solvent with water and measuring turbidity as an indicator of solubility. When mixtures are less than fully transparent, the mixture is no longer a molecular solution. Hydric solvents that are fully miscible with water at ambient temperature and shower temperature tend to work well in helping with microemulsion formation. A spectrophotometer can be used to measure miscibility by optical clarity, by measuring % light transmission at a visible wavelength of light such as 640 nm. Hydric solvent is added in increasing amounts to water, measuring optical clarity. When all mixtures are optically transparent, the solvent is fully water miscible. If some mixtures are less than transparent, the concentration of hydric solvent at the onset of turbidity is its aqueous solubility.

Perfume miscibility of a hydric solvent can be determined by mixing the solvent with a representative perfume or perfume molecule and measuring optical clarity. When the perfume-solvent mixture is less than fully transparent the mixture is no longer a molecular solution. Hydric solvent is added until past the point of optical clarity, using a spectrophotometer to measure % Transmission for the mixture at an optical wavelength. Perfume miscibility is defined as the highest percentage of hydric solvent that can be added to a perfume, based on the weight of the two, which remains optically clear, i.e., generally 100% T at 640 nm (minus a small amount of absorbance, but not scattering). Exemplary hydric solvents are at least 10%, 15%, 20%, or 40 wt% miscible in the target perfume based on total weight of the perfume-solvent mixture. When solvent is miscible with both perfume and water, surface tension between the water and perfume phases in a composition can be lower, which creates optimal conditions for the formation of a microemulsion. When miscibility with both water and perfume is even higher, i.e., as high as 100%, solvent located in a microemulsion core with perfume can rapidly migrate to the aqueous phase during product use. The abundance of additional water during cleansing thus can reduce solvent in the microemulsion perfume core, reducing its action as a perfume solvent. This increases the thermodynamic activity coefficient of the perfume allowing it to both bloom in the shower and partition into the skin to provide superior scent longevity.

Perfume miscibility of a hydric solvent can vary by perfume since each perfume has unique chemical components. Perfume miscibility can be measured for a particular solvent in a specific perfume, such as the table below demonstrates for the perfume having the components listed below and used in the first series of composition examples further below.

A perfume, perfume X, was used having these below in addition to other components, the components below were identified from the spectra, area counted and summed per the PHADD method disclosed herein. PRM (KI): Alpha pinene (940), camphene (955), myrcene (990), para-cymene (1028), d-limonene (1034), eucalyptol (1037), dihydromyrcenol (1071), alpha terpinene (1022), linalool (1107), camphor (1154), methyphenylcarbinyl acetate (1193), florol major 2 (1197), allyl amyl glycolate major (1234), linalyl acetate (1254), coranol (1275), 1H-Indene,2,3-dihydro-1,1,2,3,3-pentamethyl (1325), neryl acetate (1364), cyclemax (1427), coumarin (1449), gamma methyl ionone (1491), butylated hydroxyl toluene (1519), cashmeran (1517), methyldihydrojasmonate (1663), cis-hexenyl salicylate (1680), Iso-E Super Major (1686), Helvetolide major (1727), ambroxan major 2 (1791), galaxolide (1874).

| | Perfume X miscibility (% of solvent added) |
|---|---|
| dipropylene glycol | 100% |
| hexylene glycol | 100% |
| *PEG 300 | 57% |
| propylene glycol | 38.5% |
| 1,3-butanediol | 33.0% |
| 1,6-hexanediol | 25.1% |
| glycerine | 3.2% |

| | |
|---|---|
| * Dow Chemicals Carbowax Sentry PEG 300 average molecular weight | |

Surfactant miscibility of a hydric solvent can also be important to ability of the microemulsion to form. Preferred hydric solvents can form an optically transparent mixture when 45 parts surfactant are mixed with 30 parts hydric solvent and 25 parts water, which mixture can absorb an additional 30 parts of perfume oil while remaining optically clear.

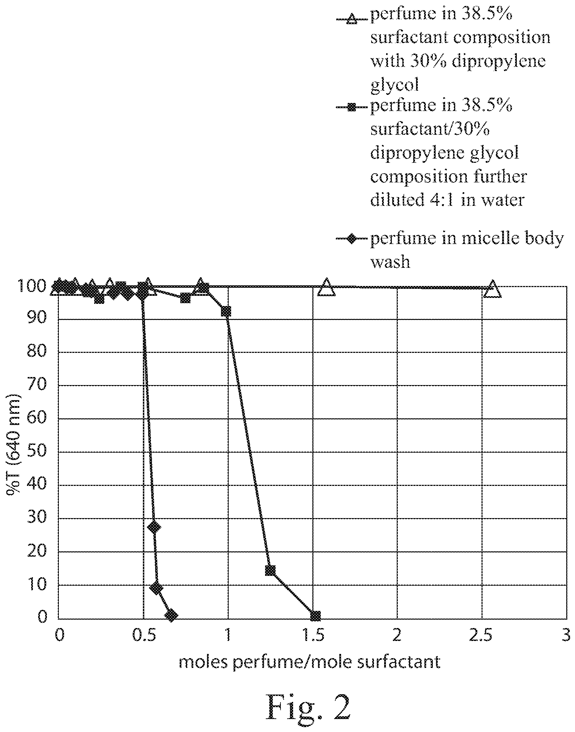

When a hydric solvent meets these aforementioned criteria, generally a transparent microemulsion can be formed in the presence of perfume and surfactant with the solvent such that the composition can absorb at least 0.5 parts of perfume oil:surfactant while retaining optical clarity. Generally, a micelle is only capable of absorbing 25% perfume oil by weight of the surfactant. Whereas microemulsion compositions can absorb 50% or 75% or 100% or more, of the weight of the surfactant, in perfume oil while retaining optical clarity (see FIG. 2, the triangle (microemulsion containing composition) versus the diamond (micellar body wash)). In some cases, it may take a day or more for the microemulsion to spontaneously form at ambient temperature, to establish the equilibrium phase behavior.

One class of a hydric solvent that is miscible with water and many perfumes is glycol. Glycols may have a mixture of isomers. One exemplary glycol class is diols where the alcohol groups are separated by no more than 2 carbons on average. Suitable glycols can include, for example, dipropylene glycol, diethylene glycol, dibutylene glycol, propylene glycol, butylene glycol, pentylene glycol, heptylene glycol, hexylene glycol, polyethylene glycol having a weight average molecular weight below 500, or combinations thereof. Glycols can include pure materials and mixtures of isomers. For example, hexylene glycol includes 1,6-hexane diol; 1,4-hexane diol; or methyl pentanediol structures having 2 alcohol groups, etc.

Hydric solvents can modify the rheological properties of the composition, particularly reducing the viscosity. It was previously discussed that concentrating a composition into a gel is one way to combat low viscosity to improve dispensing and spreading characteristics. These types of compositions often exhibit a classic x-ray diffraction pattern of a lamellar phase. However, when the level of hydric solvent is greater than 40%, by weight of the surfactant, it can be difficult to form a structured gel and thus the composition can have a much lower viscosity and is difficult to dispense from conventional body wash packages. When compositions with an exemplary rheology profile are desired, an intermediate level of hydric solvent can be used to deliver both exemplary rheology and perfume delivery properties. Thus, the hydric solvent can be from 15% to 40%, from 17% to 35%, from 20% to 30%, expressed as a weight percent of the surfactant.

Perfume is a benefit agent. Perfume benefits can be realized at different time points for cleansing compositions. Perfume in the package headspace can be important to select a product at the time of purchase. Perfume scent during cleansing, upon introduction of modest amounts of water, such as for example 3 parts of water per part composition (i.e., a 3:1 dilution ratio), provides a benefit during skin cleansing. During skin cleansing, some perfume can partition into the outer layers of the skin or hair, which can provide a scented skin or hair benefit for a period of time after cleansing, called scent longevity. A governing property for both scent bloom and longevity is the activity coefficient of the perfume molecules, which is a thermodynamic term. Perfume molecules exhibit their maximum vapor pressure only when they are pure. Diluted perfume molecules, whether diluted by surfactant in a micelle, organic solvent, water, etc., exhibit less than their pure vapor pressure. The amount of perfume in a headspace over a composition, diluted composition, or over the skin or hair can be measured analytically, as described in the methods section below. Benefits in initial fragrance intensity, bloom, or longevity can be demonstrated by comparing performance of the compositions before, during, and, or after a skin or hair cleansing event, compared to conventional body wash or shampoo compositions.

In addition to being a benefit agent, perfume is an oil and therefore can be a direct contributor to formation of phases responsible for its activity coefficient (as noted above), and therefore to scent bloom and longevity benefits. As discussed above, perfume oil can generally be added into micelle surfactant mixtures only to 0.25 weight fraction of the surfactant before it phase separates, whereas diluted cleansing compositions described herein can hold at least 0.5 parts perfume:surfactant, or even 0.75 parts perfume:surfactant, or even more, while remaining transparent, including water diluted compositions. The ability to hold large amounts of perfume in this manner while remaining transparent, isotropic and low viscosity is an indication the diluted composition is a microemulsion phase and is suitable for enhanced perfume benefits.

Perfume can be a carrier for non-scented, hydrophobic additives. Additives which are at least 5 wt%, or at least 10 wt%, or at least 20 wt% miscible with perfume may be employed to increase delivery of the additives to the skin or hair. Any additive which provides a benefit to the skin or hair or the skin environment (e.g., the skin microbiome) may be employed. The additive may provide a direct or indirect benefit, such as antibacterial, antihyperproliferative, antiinflammatory, chelation, pH regulation, antifungal, antiviral, control of disorders such as acne, atopic dermatitis, eczema, dermatitis, dandruff, antiaging, antiwrinkle, age spot reduction, sunscreen, hydration, moisturization, or any other skin benefit. An advantage of the present compositions is enhanced additive delivery to the skin or hair during cleansing. A further benefit is reduction in activity coefficient of the additive by dilution with perfume is transient due to subsequent evaporation of the perfume on the skin, which increases the thermodynamic activity of the additive after its delivery to the skin.

In addition, some compositions may form microemulsions but perform poorly for lathering and hence cleaning, which are important features for consumers. Compositions which effectively deliver perfume as described above, can also have consumer acceptable lather properties. Lather can be measured in accordance with the Cylinder Method described below. Compositions may have a lather volume of 300 mL, 400 mL, 500 mL, 600 mL, 700 mL, or more. Compositions may have a lather density of 0.03 g/cc, 0.04 g/cc, 0.05 g/cc, 0.055 g/cc, 0.06 g/cc, 0.065 g/cc, or more. Compositions may have a lather mass of 20 g, 25 g, 30 g, 35 g, 40 g, 45 g, or more.

In accordance with the above, a cleansing composition comprises a surfactant, a hydric solvent, perfume, and water. Additionally, optional ingredients may also be included as noted herein, for example, preservatives, thickeners, hydrophobic oils, pH modifiers, additives, soap, etc. The cleansing composition is not in the form of a ringing gel. The cleansing composition can be in the form of a microemulsion or may contain a microemulsion phase. At least a portion of the cleansing composition may become a microemulsion upon dilution with water of 2:1 or 3:1 by weight (water:composition) to 10:1 by weight (water:composition).

### A. Surfactant

A rinse-off cleansing composition includes surfactant. Surfactants can provide a cleaning benefit, lather properties, and rheology properties to the compositions. The surfactant may be a single surfactant or a combination of multiple surfactants. In addition, a surfactant may be branched, linear, or a combination thereof. A composition may comprise from 35% to 85%, from 35% to 80%, from 35% to 70%, from 35% to 60%, or from 40% to 50%, by weight of the composition, of total surfactant. The previous weight percentages of surfactant in the composition include primary surfactant and any cosurfactant.

The surfactant may be anionic, zwitterionic, amphoteric, nonionic, or a combination thereof. The surfactant may include a first surfactant and a cosurfactant. The rinse-off cleansing composition may include a first surfactant at a level of from 16% to 85%, from 25% to 60%, from 35% to 45%, from 25% to 45%, or from 30% to 40%, by weight of the composition. The first surfactant can be, for example, anionic.

The anionic surfactant can be linear or branched. The anionic surfactant may contain any counterion such as sodium, potassium, ammonium, triethanolamine, etc. The hydrocarbon chain can be an olefin or be branched or linear or cyclic, such as alkyl benzenes, and generally has between 10 and 20 carbons or 12 to 16 carbons. The anionic surfactant can comprise ethylene oxide groups, such as one EO, or two EO, or three EO, e.g., and can be a sulfate, sulfonate or carboxylate, including acidic sulfonates such as sulfosuccinates. Some exemplary anionic surfactants include a sulfate, an alkyl ether sulfate, an alkyl ether sulfate with 0.5 to 5 ethoxylate groups, sodium trideceth -2 sulfate, or a combination thereof.

Suitable anionic surfactants can include, for example, sodium trideceth sulfate and sodium laureth sulfate. These materials can have varying levels of ethoxylation. Thus, the levels of ethoxylation are represented by an (n), for example, sodium trideceth-n sulfate. n can range from 0.5 to 5. Some exemplary anionic surfactants are sodium trideceth-2 sulfate, sodium trideceth-3 sulfate, sodium laureth-1 sulfate, sodium laureth-2 sulfate, sodium laureth-3 sulfate, or combinations thereof. The anionic surfactant can be a branched surfactant comprising sodium trideceth-2 sulfate, sodium trideceth-3 sulfate, or a combination thereof. In one example, the cleansing composition comprises from 35% to 45%, by weight of the composition, of sodium trideceth-2 sulfate.

The rinse-off cleansing composition may include from 1% to 20%, from 2% to 10%, from 5% to 10%, or from 5% to 8%, by weight of the composition, of a cosurfactant. The cosurfactant may be, for example, zwitterionic surfactant, amphoteric surfactant, nonionic surfactant, or a combination thereof. Suitable amphoteric or zwitterionic surfactants can include those described in U.S. Patent No. 5,104,646 and U.S. Patent No. 5,106,609.

Additional amphoteric detersive surfactants suitable for use in the rinse-off cleansing compositions can include those surfactants broadly described as derivatives of aliphatic secondary and tertiary amines in which an aliphatic radical can be straight or branched chain and wherein an aliphatic substituent can contain from 8 to 18 carbon atoms such that one carbon atom can contain an anionic water solubilizing group, e.g., carboxy, sulfonate, sulfate, phosphate, or phosphonate. Examples of compounds falling within this definition can be sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Patent No. 2,658,072, N-higher alkyl aspartic acids such as those produced according to the teaching of U.S. Patent No. 2,438,091, and products described in U.S. Patent No. 2,528,378. Other examples of amphoteric surfactants can include sodium lauroamphoacetate, sodium cocoamphoacetate, disodium lauroamphoacetate disodium cocodiamphoacetate, and mixtures thereof. Amphoacetates and diamphoacetates can also be used.

Zwitterionic surfactants suitable for use in the rinse-off cleansing compositions are well known in the art, and include those surfactants broadly described as derivatives of aliphatic quaternary ammonium, phosphonium, and sulfonium compounds, in which aliphatic radicals can be straight or branched chains, and wherein an aliphatic substituent can contain from 8 to 18 carbon atoms such that one carbon atom can contain an anionic group, e.g., carboxy, sulfonate, sulfate, phosphate, or phosphonate. Other zwitterionic surfactants can include a betaine, like an alkyl betaine or an alkyl amidopropyl betaine, like cocamidopropyl betaine. The composition may comprise a betaine, an alkyl amidopropyl betaine, a cocamidopropyl betaine, or a combination thereof.

Nonionic surfactants suitable for use can include those selected from the group consisting of alkyl ethoxylates, alkyl glucosides, polyglucosides (e.g., alkyl polyglucosides, decyl polyglucosides), polyhydroxy fatty acid amides, alkoxylated fatty acid esters, sucrose esters, amine oxides, or mixtures thereof. Some exemplary nonionic surfactants can include cocamide monoethanolamine, decyl glucoside, or a combination thereof.

### B. Perfume

A rinse-off cleansing composition includes a perfume. A composition may comprise from 4% to 25%, from 4% to 30%, from 5% to 20%, from 6% to 15%, from 6% to 30%, from 7% to 25%, from 8% to 20%, from 8% to 15%, or from 4% to 15%, by weight of the composition, of perfume.

Perfume may include solvents such as triethyl citrate, isopropyl myristate, dipropylene glycol, or others, to help, for example, with the miscibility of the perfume molecules with each other or to reduce cost. Generally these perfume solvents provide minimal or negligible effects on surfactant compositions as a whole due to the low amount of perfume in the total composition and the amount of solvent in a perfume can be ignored. However, when solvent in the perfume accounts for more than 5 wt% of the total hydric solvent in the cleansing composition, it should be accounted for. For example, when a perfume containing 10% hydric solvent is added to a cleansing composition at a level of 10 wt.% and the composition has 10 wt.% of added hydric solvent, the 1 wt. % of hydric solvent from the perfume accounts for a 9% increase in hydric solvent in the cleansing composition (1/11). Since this is more than a 5% change in the hydric solvent in the composition, it can be important. In this case, hydric solvent from the perfume is added (mathematically) to the hydric solvent from other sources added to the composition; and perfume is considered to comprise only the scented molecules and not the solvent, which is subtracted from the wt% perfume in the composition.

In addition, the weight ratio of perfume to surfactant can impact the ability of the composition to provide an enhanced fragrance benefit. Without being limited by theory, it is believed at least some of the perfume benefits, like bloom and residual scent are derived from an abundance of perfume on the basis of its relation to the surfactant due at least in part to the interaction of the perfume with surfactant as the composition is diluted. Perfume is soluble in surfactant micelles only to 25% by weight of the surfactant. Above this level, the composition can become unstable unless steps are taken to form a phase to accept the abundance of perfume. However, forming those phases for stability of the perfume circles the composition back to where the perfume is bound within the composition and difficult to release. As such, a rinse-off cleansing composition comprises from 2%, 4%, 5%, 8%, 10 % 12%, 15%, 20%, 25%, 30% 35%, 40%, 50%, 70%, to 15%, 30%, 40%, 50%, 60%, 70%, 90%, by weight of the surfactant, of perfume.

Perfumes generally contain a broad range of perfume molecules (PRM) having diverse properties. It is an oversimplification to suggest all of the perfume is in a particular location, like in the core of a microemulsion. The real picture is more complex, with perfume molecules in dynamic equilibrium and structures such as micelles and microemulsions can be percolating. Further, some perfume molecules may favor being among surfactant tails or even in the aqueous phase instead of the microemulsion core. In short, all perfume molecules within a perfume mixture do not behave identically. Certain generalizations are useful to explain observed behaviors without inferring that all molecules in a perfume behave identically. For our purposes, a broad array of perfume molecules in a perfume mixture are analyzed by averaging or summing their performance.

Certain perfume features may also impact perfume benefits, such as the proportion of perfume molecules within a volatility or molecular weight range. In general, Kovats Index (KI) is a useful parameter to differentiate perfume molecules. Perfume molecules having KI less than 1100 can be considered high blooming molecules; those having KI greater than 1400 can be considered high skin partitioning molecules; and those between (KI of 1100-1400) can be considered middle perfume notes which generally favor neither bloom nor skin partitioning, but contribute to some extent in both.

Perfume can be tailored to enhance features of the compositions. For example, while the compositions, including diluted compositions during use, can have a high activity coefficient, perfume molecules may selectively evaporate to enhance bloom or partition into the skin depending on their individual vapor pressure. It has surprisingly been discovered that the weight percentage of middle notes can impact the fragrance expression of the composition for the initial scent, for bloom and delivery on the skin. Particularly, better expression of the perfume is accomplished when the weight percentage of middle notes is restricted. For example, the composition may comprise a perfume, wherein the weight percentage of the perfume components having a Kovats Index of 1100 to 1700 comprises from 0% to 70%, from 5% to 50%, from 5% to 30%, or from 5% to 20%, by weight of the perfume.

In addition, it has also been discovered that the weight percentages of the perfume raw materials in a perfume composition can provide a strong rheological effect on the rinse-off cleansing composition. The wt% proportion of low, mid and high KI materials in the perfume impacts the elastic and viscous modulus of the composition as well as the viscosity. In general having a greater proportion of low KI materials results in a reduction in G' and G" and a lower tan delta (ratio of G"/G'). The following models of G' and G" were developed based on samples containing various proportions of low, mid and high KI materials and is a demonstration of the impact of KI on rheological properties for an exemplary concentrated body wash composition. G' = 637.5 - (1.118^{∗}wt% of Low KI Materials in a perfume) + (2.879^{∗}wt% proportion of Mid KI Materials in a perfume) and G" = 7.510 + (0.4056^{∗}wt% of Mid KI Materials in a perfume) + (0.6140^{∗}wt% of High KI materials in a perfume).

### C. Solvent

A rinse-off cleansing composition includes a solvent. A rinse-off cleansing composition may comprise from 6% to 20%, from 7% to 18%, from 8% to 16%, from 9% to 15%, or from 10% to 14%, by weight of the composition, of the solvent.

The solvent can be a hydric solvent. The hydric solvents include dipropylene glycol (a glycol ether), diethylene glycol, dibutylene glycol, hexylene glycol, butylene glycol, pentylene glycol, heptylene glycol, propylene glycol, a polyethylene glycol having a weight average molecular weight below 500, or a combination thereof. One example of a polyethylene glycol is PEG 300. Isomers are included in the generally descriptive solvents listed, for example, butylene glycol is meant to included 1,2-butanediol and 1,3-butanediol and 1,4-butanediol. When solvents are solid in the pure form (e.g., 1,6-hexanediol), they can be melted during the making process and are effective hydric solvents. The composition comprises at least 5%, 6%, 8%, 10%, or 12%, to 20%, 25%, 30%, 35%, or 40%, by weight of the composition, of hydric solvent.

In addition, a cleansing composition may comprise from 7%, 10%, 12%, 14%, 17%, 20%, 25%, 30%, 40%, 50%, or 60%, to 40%, 50%, or 60%, or any combination thereof, by weight of the surfactant, of hydric solvent. For example, one exemplary cleansing composition will have 6%, by weight of the composition, of hydric solvent, and 44.5%, by weight of the composition, of surfactant. Hydric solvent levels can be expressed as a percent of the surfactant because the solvent molecules can engage with the surfactant molecules.

An intermediate level of hydric solvent can be used to deliver both a combination of exemplary rheology and perfume delivery properties. Thus, the hydric solvent can be from 15% to 40%, from 17% to 35%, from 20% to 30%, expressed as a weight percent of the surfactant.

A solvent may also comprise a non-hydric solvent. Examples of non-hydric solvents include propylene carbonate, butanol, pentanol, hexanol, propylene glycol ethers, butyl butanoate, propyl propanoate, isopropyl propanoate, or a combination thereof. One example of a propylene glycol ether is propylene glycol monomethylether. The non-hydric solvent may comprise less than 25%, 20%, 15%, 10% or 5% by weight of the solvent.

### D. Water

A rinse-off cleansing composition includes water. Water may come in with other components or may be added as free water. A rinse-off cleansing composition may comprise from 2% to 57%, from 5% to 55%, from 10% to 50%, from 15% to 50%, or from 25% to 45%, by weight of the composition, of water.

In addition, the total weight percent of water and solvent can be important in the composition since this defines the amount of solvent phase in which the microemulsion or surfactant structures are distributed. The total amount of solvent phase (approximately, the additive inverse generally of the surfactant level) is a key driver of surfactant phases due to proximity of surfactants. Thus, the composition may comprise from 8% to 75%, from 15% to 70%, from 25% to 65%, from 30% to 61%, by weight of the composition, of the combination of water and solvent.

### E. Rheology - Viscoelasticity and Viscosity

The rheological properties of rinse-off cleansing compositions can be characterized by viscoelastic parameters and a viscosity. The rheology of a composition can be defined by its G' and G" values, relating to the composition's structure. G' and G" are measured in accordance with the rheological properties method discussed herein. G' and G" describe a cleansing compositions elastic and viscous response to applied stress, characterizing how the material acts when dispensed from a bottle, sitting on the consumers implement or hand, and how a product spreads on application. It also impacts a consumer's perception of the product, for instance products with low G' values flow too readily in use and are associated in consumer perception and can be perceived as dilute. Conversely products with a high G' are associated in consumer perception with concentrated personal cleansing products. The cleansing composition may have a G' at 1 Hz of 25 Pa to 3000 Pa; from 50 Pa to 2500 Pa, from 100 Pa to 1500 Pa, or from 150 Pa to 1000 Pa. The cleansing composition may have a G" at 1 Hz of 20 Pa to 250 Pa; from 35 Pa to 200 Pa, from 40 Pa to 150 Pa, or from 50 Pa to 100 Pa.

In addition, the cleansing composition should have a viscosity sufficient to allow it to be dispensed from a package onto an implement or directly onto the skin. The viscosity of a rinse-off cleansing composition is measured in accordance with the rheological properties method discussed herein. The cleansing composition may have a viscosity at 0.10 1/sec of 10 PaS to 1200 PaS; from 20 PaS to 1000 PaS, from 30 PaS to 500 PaS, or from 40 PaS to 300 PaS. The cleansing composition may have a viscosity at 10 1/sec of 1 PaS to 30 PaS; from 1 PaS to 20 PaS, from 1 PaS to 15 PaS, or from 1 PaS to 10 PaS.

Compositions can also be highly shear thinning, having a viscosity ratio of less than 0.20, or 0.10, or even less than 0.05, which is the ratio of the viscosity at 10 1/sec divided by the viscosity at 0.10 1/sec.

### F. Preservatives

Liquid cleansing compositions often have a high water activity (i.e. 0.95 or more). Water activity describes the availability of water within a composition to support various chemical and biological processes requiring water. Compositions with high water activity can allow growth of microorganisms and therefore generally utilize preservatives. For example, bacteria can grow at a water activity of 0.90 or above and fungus can grow at a water activity of 0.70 or above. Below these water activities, microorganisms generally dehydrate and die.

The rinse-off cleansing compositions as noted herein can have a low water activity, less than 0.90. This low water activity allows the compositions to naturally resist the growth of microorganisms and thus utilize minimal or even no, preservative. In addition, the use of high levels (5 wt. % or more) of glycols, like dipropylene glycol, can also help to prevent the growth of microorganisms and further support a composition which needs minimal or even no, preservative.

### G. Hydrophobic Oils

The rinse-off cleansing composition may comprise a hydrophobic oil. Hydrophobic oil can help form a microemulsion phase due to low solubility in the palisade layer of micelles, to further enhance bloom and deposition on skin. The rinse-off cleansing composition may comprise from 0% to 25%, from 2% to 20%, or from 3% to 15% by weight of the composition, of a hydrophobic oil. Exemplary hydrophobic oils can include, for example, isopropyl myristate, isostearyl isostearate, behenyl behenate, triglycerides such as soybean oil, hydrocarbon such as mineral oil, or combinations thereof.

### H. Additives

The rinse-off cleansing composition may comprise an additive. Additives are materials that are at least partially soluble in the perfume. It is believed that additives which are at least partially soluble in the perfume will also see a deposition benefit. Additives which are at least 5 wt%, or at least 10 wt%, or at least 20 wt% miscible with perfume may be employed to increase delivery of the additives to the skin or hair. Some examples of classes of material that can be soluble in the perfume are skin actives, vitamins, antibacterials, antifungals, chelants, or combinations thereof.

Examples of skin actives which can be included are sunscreens; anti-acne medicaments; antioxidants; skin soothing agents, skin healing agents; essential oils, skin sensates, anti-wrinkle medicaments, or mixtures thereof. Some examples of skin soothing agents can include, for example, aloe vera, allantoin, bisabolol, dipotassium glycyrrhizinate, or combinations thereof.

Examples of vitamins which can be included are Vitamin A (e.g., beta carotene, retinoic acid, retinol, retinoids, retinyl palmitate, retinyl proprionate, etc.), Vitamin B (e.g., niacin, niacinamide, riboflavin, pantothenic acid, etc.), Vitamin C (e.g., ascorbic acid, etc.), Vitamin D (e.g., ergosterol, ergocalciferol, cholecalciferol, etc.), Vitamin E (e.g., tocopherol acetate, tocopherol nicotinate, etc.), Vitamin K (e.g., phytonadione, menadione, phthiocol, etc.), or combinations thereof.

Examples of antibacterials and/or antifungals which can be included are glycolic acid, lactic acid, phytic acid, N-acetyl-L-cysteine, phenoxyethanol, phenoxypropanol, phenoxyisopropanol, zinc pyrithione, octopirox (piroctone olamine), climbazole, ketoconazole, thymol, terpineol, essential oils, or combinations thereof.

Examples of chelants which can be included are 2-aminoethyl phosphoric acid (AEP), N-phosphonomethyl aminodiacetic acid (PMIDA), 1-hydroxyethane-1,1-diphosphonic acid (HEDP), amino tris(methylene phosphonic acid) (ATMP), ethylenediamine tetra(methylene phosphonic acid) (EDTMP), diethylenetriamine penta(methylene phosphonic acid) (DTPMP), phytic acid, nitrilotrimethylene phosphonic acid (NIP), 2-hydroxypyridine oxide (HPNO), or combinations thereof.

The rinse-off cleansing composition may comprise from 1% to 20%, from 2% to 10%, or from 3% to 8%, by weight of the composition, of an additive.

### I. Thickeners

The rinse-off cleansing composition may comprise from 0.1% to 4% by weight of the composition of a thickener. Preferred thickeners are hydrophilic such as cellulose derivatives, hydrophobically modified celluloses, starches and starch derivatives, polyacrylates including hydrophobically modified polyacrylates and polyacrylamides, bacterial polymers such as xanthan gum, tree and plant gums such as guar, insoluble thickeners such as cellulose.

### J. Soap

Rinse-off cleansing compositions as described herein may also comprise soap.

### K. Packaging

Compositions can be dispensed from a squeezable package with an orifice, such as a conventional body wash or shampoo package. The package can be a compact package, i.e., contain less than 250 ml, or 200 ml, or 150 ml of volume to signal the contents are concentrated. The shear thinning compositions can be dispensed from a package with a slit valve orifice or other flexible orifice, which is generally cut from a silicone elastomeric material and inserted into an orifice housing. When the composition has a low viscosity, less than 0.25 PaS at 10 1/sec, it can be dispensed from a foaming package such as a pump foamer. Compositions can also be dispensed from liquid pump packages.

### L. Methods

In addition to the compositional elements and parameters noted above, it is believed there are also some inventive benefits and/or uses to the compositions which are set out as methods below. For the sake of brevity, all of the compositional elements and parameters noted above are not repeated herein, but can be used within the methods where relevant.

A method of enhancing fragrance longevity on skin of a rinse-off cleansing composition, comprising, combining: a) from 35% to 85%, by weight of the composition, of surfactant; b) from 4% to 30%, by weight of the composition, of a perfume, wherein the weight percent of perfume is from 12% to 40%, by weight of the surfactant; c) from 6% to 20%, by weight of the composition, of a hydric solvent, wherein the hydric solvent comprises dipropylene glycol, diethylene glycol, dibutylene glycol, hexylene glycol, butylene glycol, pentanediol, heptanediol, propylene glycol, a polyethylene glycol having an average molecular weight below 500, or a combination thereof, preferably dipropylene glycol, and wherein the weight percent of the hydric solvent is from 16% to 24%, by weight of the surfactant; and d) from 2% to 57%, by weight of the composition, of water, to form the cleansing composition; wherein the rinse-off cleansing composition is not a ringing gel; wherein the rinse-off cleansing composition has a G' at 1 Hz of 25 Pa to 3000 Pa, and wherein the composition has a total GCMS count higher than that of a control where the solvent is replaced with water, when the total GCMS count is measured in accordance with the PHADD method at zero dilution. The composition may have a GCMS total count of 10% more than the control, 15%, 20%, 25%, 50%, 75%, 100%, 150%, 200%, 250%, or even 300% more than the control.

### Examples

All inventive and comparative samples are prepared by weighing the components together into a Speedmixer pot, stirring by hand briefly to homogenize the fluids, and then speedmixing for 60 seconds at 2750 rpm.

| | Comp. A | Ex. 1 | Ex. 2 | Ex. 3 | Ex. 4 | Ex. 5 |
|---|---|---|---|---|---|---|
| Sodium trideceth-2 sulfate | 38.5 | 38.5 | 38.5 | 37.9 | 36.9 | 40.3 |
| Cocamidopropyl betaine | 6.0 | 6.0 | 6.0 | 5.9 | 5.7 | 4.2 |
| Dipropylene glycol | 0 | 7.0 | 10.6 | 12.6 | 14.6 | 14.6 |
| Water | qs | qs | qs | qs | qs | qs |
| Citric acid | 0.5 | 0.5 | 0.5 | 0.5 | 0.5 | 0.5 |
| perfume | 9.0 | 9.0 | 9.0 | 9.0 | 9.0 | 9.0 |
| preservatives | 0.5 | 0 | 0 | 0 | 0 | 0 |
| | | | | | | |
| G' at 1 Hz (Pa) | 2,340 | 1,500 | 726 | 516 | 241 | 497 |
| G" at 1 Hz (Pa) | 203 | 227 | 93 | 56.5 | 51.1 | 75 |
| Viscosity at 1 sec-1 (PaS) | 93.3 | 38 | 16.1 | 10.8 | 4.8 | 10.9 |
| Viscosity ratio | 0.026 | 0.023 | 0.016 | 0.023 | 0.033 | 0.018 |
| | | | | | | |
| Total surfactant | 44.5 | 44.5 | 44.5 | 43.8 | 42.6 | 44.5 |
| Hydric solvent as percent of surfactant | 0% | 15.7% | 23.8% | 28.8% | 34.3% | 32.8% |
| Perfume as percent of surfactant | 22.5% | 22.5% | 22.5% | 22.8% | 23.5% | 22.5% |

The above examples were subjected to the PHADD test and the counts are listed in the table directly below. The "dilutions X:X and lower" or "dilutions between X:X and X:X) is the average of the absolute counts in the noted dilution range. This data is graphically represented in FIG. 1. The data below and in Fig. 1 shows as DPG is increased in the compositions, a modest increase (30-40%) in perfume in the headspace over the compositions results when between 7 and 10.6 wt% dipropylene glycol is added, based on the weight of the composition; and when 12 - 19 wt% dipropylene glycol is added the perfume counts in the headspace over the composition increase to between 16 and 21 million counts, which is 2-fold to 3-fold the headspace counts for the composition without hydric solvent. During the aqueous dilution, when the composition contains greater than 12 wt% dipropylene glycol, perfume counts remain consistently higher than control, in some cases by more than 3-fold. When the composition contains 10.6 wt% dipropylene glycol, the dilution behavior is quite extraordinary in that between a 1.5:1 and 2:1 dilution, there is a dramatic increase in perfume in the headspace. At 7 wt% dipropylene glycol, the increased headspace response compared to the control with no DPG is substantial but less dramatic. The table below shows compositions with dipropylene glycol become transparent at 42 degrees C (an average shower temperature) at relatively low dilution ratios with water, transparency suggesting the compositions can be microemulsions above the dilution shown. However, they can also be transparent micelles, so the perfume measurements directly indicate the benefit relative to the control. In addition, microemulsions can also contain amounts of hydric or non-hydric solvent in the core, associated with the perfume, reducing its activity coefficient. Therefore, in theory, not all microemulsions can increase perfume activity coefficient relative to a micelle, because of the solvent interaction. These results demonstrate the microemulsions provide significantly enhanced perfume benefits when the hydric solvent dipropylene glycol is used at between 7 wt% and 19 wt% of the composition.

Perfume Count in Headspace measured in accordance with PHADD

| | Comp. A | Ex. 1 | Ex. 2 | Ex. 3 | Ex. 4 | Ex. 5 |
|---|---|---|---|---|---|---|
| Undiluted (absolute counts) | 7,035,158 | 9,005,372 | 9,943,265 | 21,004,851 | 20,239,653 | 16,263,229 |
| Dilutions 1:1 and lower including undiluted (absolute counts) | 7,035,158 | 10,205,115 | 11,063,020 | 22,150,969 | 20,239,653 | 17,549,982 |
| Dilutions between 2:1 and 5:1 inclusive (absolute counts) | 9,525,565 | 10,184,234 | 25,460,606 | 23,817,460 | 22,888,702 | 16,653,508 |
| Ratio to control with nil DPG, undiluted | 1 | 1.28 | 1.41 | 2.99 | 2.88 | 2.31 |
| Ratio to nil DPG control, dilutions 1:1 and lower | 1 | 1.45 | 1.57 | 3.15 | 2.88 | 2.49 |
| Ratio to nil DPG control, dilutions 2:1 to 5:1 inclusive | 1 | 1.07 | 2.67 | 2.50 | 2.40 | 1.75 |
| | | | | | | |
| Clarity commencing at dilution at 42°C at this dilution level | 2:1 | | 0.9:1 | 0.8:1 | 0.5:1 | 0.8:1 |

| | Comp. B | Ex. 6 | Ex. 7 | Ex. 8 | Ex. 9 |
|---|---|---|---|---|---|
| Sodium trideceth-2 sulfate | 38.5 | 38.5 | 38.5 | 38.5 | 38.5 |
| Cocamidopropyl betaine | 6.0 | 6.0 | 6.0 | 6.0 | 6.0 |
| Dipropylene glycol | 9.6 | 9.6 | 9.6 | 9.6 | 9.6 |
| Water | qs | qs | qs | qs | qs |
| Citric acid | 0.5 | 0.5 | 0.5 | 0.5 | 0.5 |
| perfume | 2.0 | 4.0 | 6.0 | 8.0 | 10.0 |
| preservatives | 0 | 0 | 0 | 0 | 0 |
| | | | | | |
| G' at 1 Hz (Pa) | 341 | 432 | 514 | 455 | 817 |
| G" at 1 Hz (Pa) | 48 | 61 | 75 | 62 | 75 |
| Viscosity at 1 sec⁻¹ (PaS) | 7.0 | 10.1 | 10.9 | 9.9 | 13.4 |
| Viscosity ratio | 0.037 | 0.020 | 0.019 | 0.023 | 0.025 |
| Total surfactant | 44.5 | 44.5 | 44.5 | 44.5 | 44.5 |
| Hydric solvent as percent of surfactant | 21.6 | 21.6 | 21.6 | 21.6 | 21.6 |
| Perfume as percent of surfactant | 4.5 | 9.0 | 13.5 | 18.0 | 22.5 |

The above compositions compare varying levels of perfume within a cleansing composition with hydric solvent. The perfume counts for these compositions measured in accordance with PHADD are listed directly below. As can be seen below, the level of perfume can have an impact on the headspace counts both before and after dilution.

| Perfume count in headspace | | | | | |
|---|---|---|---|---|---|
| Undiluted (absolute counts) | 10,977,417 | 23,647,482 | 31,214,245 | 40.124,155 | 43,428,087 |
| Dilutions 1:1 and lower including undiluted (absolute counts) | 13,102,290 | 26,197,063 | 34,274,057 | 43,9455,804 | 49,582,089 |
| Dilutions between 2:1 and 5:1 inclusive (absolute counts) | 15,997,105 | 28,731,188 | 41,179,435 | 42,000,908 | 49,582,089 |

The compositions below are examples of cleansing compositions with varying solvents. The perfume counts for these compositions measured in accordance with PHADD are listed directly below.

| | Ex. 10 | Ex. 11 | Ex. 12 | Ex. 13 | Ex. 14 | Ex. 15 | Comp. C |
|---|---|---|---|---|---|---|---|
| Sodium trideceth-2 sulfate | 33.5 | 33.5 | 33.5 | 33.5 | 33.5 | 33.5 | 33.5 |
| Cocamidopropyl betaine | 6.0 | 6.0 | 6.0 | 6.0 | 6.0 | 6.0 | 6.0 |
| Dipropylene glycol | 12 | | | | | | |
| Hexylene glycol | | 12 | | | | | |
| 1,6-hexanediol | | | 12 | | | | |
| Propylene glycol | | | | 12 | | | |
| 1,3-butanediol | | | | | 12 | | |
| PEG 300 | | | | | | 12 | |
| Glycerin | | | | | | | 12 |
| Water | qs | qs | qs | qs | qs | qs | qs |
| Citric acid | 0.5 | 0.5 | 0.5 | 0.5 | 0.5 | 0.5 | 0.5 |
| *perfume | 10.0 | 10.0 | 10.0 | 10.0 | 10.0 | 10.0 | 10.0 |
| preservatives | 0 | 0 | 0 | 0 | 0 | 0 | 0 |
| | | | | | | | |
| Total surfactant | 39.5 | 39.5 | 39.5 | 39.5 | 39.5 | 39.5 | 39.5 |
| Hydric solvent as a weight percent of surfactant | 30.4 | 30.4 | 30.4 | 30.4 | 30.4 | 30.4 | 30.4 |
| Perfume as weight percent of surfactant | 25.3 | 25.3 | 25.3 | 25.3 | 25.3 | 25.3 | 25.3 |

| Perfume count in headspace | | | | | | | |
|---|---|---|---|---|---|---|---|
| Undiluted (absolute counts) | 20,663,074 | 16,080,765 | 22,625,562 | 17,312,931 | 17,339,118 | 18,218,131 | 20,663,051 |
| Dilutions 1:1 and lower including undiluted (absolute counts) | 22,253,452 | 17,970,750 | 20,668,047 | 18,558,549 | 18,363,252 | 17,914,634 | 19,514,042 |
| Dilutions between 2:1 and 5:1 inclusive (absolute counts) | 21,216,488 | 19,200,632 | 18,654,679 | 18,683,788 | 18,097,084 | 17,724,486 | 21,768,735 |

| | | | | | | | |
|---|---|---|---|---|---|---|---|
| * perfume having the same components as previous examples was used. | | | | | | | |

### Example: Delivery of perfume to skin from a concentrated cleansing composition

Ex. 16 was used to wash the forearm of 2 volunteers. Their opposing forearm was washed using a control micelle body wash. The perfumes used were the same in the example and the control and the dose used to wash each arm provided the same amount of perfume exposure to each arm. The headspace over the skin was evaluated 5 minutes after washing using the PSHAM method. The example composition provided an average headspace abundance over the skin of 5.46 for one volunteer and 2.45 for the second volunteer (446% and 145% increase), or an average 3.95 ratio of perfume over the skin relative to the control. Abundance of individual perfume molecules generally increased with Kovats Index but was broadly distributed, with more than half the molecules having an abundance of more than 2.

| Ex. 16 | Weight % |
|---|---|
| Sodium trideceth-2 sulfate | 36.1 |
| Cocamidopropyl betaine | 6.2 |
| Dipropylene glycol | 12.6 |
| Water | qs |
| Citric acid | 0.666 |
| perfume | 10.0 |
| Misc. | 0.533 |
| | |
| Total surfactant | 42.3 |
| Hydric solvent as percent of surfactant | 29.8 |
| Perfume as percent of surfactant | 23.6 |

The perfume used in Ex. 16 comprised a mixture of beta-gamma hexenol, cis hexenyl formate, para cresyl methyl ether, d-limonene, isoamyl butyrate, linalool, benzyl acetate, methyl salicylate, pomarose major, citronellol, allyl amyl glycolate, carvone, undecavertol, benzyl acetate, anisic aldehyde, coranol, hydroxycitronellal, phenylethyldimethyl carbinol, heliotropin, linalyl isobutyrate, delta damascene, florhydral, beta naphthol methyl ether, dodecylnitrile, beta ionone, polysantol, citronellyl butyrate, lilial, tobacarol, hivernal, methyldihydrojasmonate, and ambroxan.

### Longevity Example

Fragrance longevity was evaluated by the PSHAM method at two time points, initial and 3.5 hours after washing, for 2 subjects. A composition was prepared having the following ingredients in wt% of the total composition. Sodium trideceth-2 sulfate 36.1, Cocamidopropyl betaine 6.2, Dipropylene glycol 12.6, Citric acid 0.50, Perfume 10.0, Dye and preservative 0.53, Water qs. 1.5 grams of the composition, which is a recommended product dose for consumers in a consumer study, was added to a wetted puff and the arms washed, rinsed, dried, and evaluated per the PSHAM procedure at an initial time point. A second comparative composition was prepared having 10.8% sodium laureth-3 sulfate, 1.2% cocamidopropyl betaine, 2.5 % sodium chloride, 1.0% perfume, 1% misc. dyes and perfume, qs water. The comparative composition was dosed using 10 ml into a puff and subjects' opposite arms washed and headspace over the skin evaluated in the same manner as above for the inventive composition. Subjects were allowed to resume normal activity, then returned after 3.5 hours for a subsequent evaluation of perfume in headspace over the skin (i.e., without a subsequent wash step, to determine residuality of the perfume from the wash). The following results were obtained.

| | Composition of Longevity Example | Control composition | Abundance (ratio to control) |
|---|---|---|---|
| PSHAM results at initial time point (total GCMS counts) | 105,877,650 | 50,740,090 | 2.09 |
| PSHAM results at 3.5 hour time point | 7,097,315 | 5,875,857 | 1.21 |

### Test Methods

### a) G' and G" Test Method

To measure the viscoelastic properties of a personal care composition, the viscous (G") and elastic (G') moduli, use a rheometer such as a AR G2 Rheometer (TA Instruments, DE, USA) with 1 degree cone upper geometry with a diameter of 40 mm and flat plate lower geometry with Peltier heating/cooling to control temperature. Place approximately 1 gram of personal care composition onto the lower test geometry and lower the upper geometry into position, lock the geometry and wipe away excess composition to create an even surface around the edge of the geometry. Conduct the oscillatory test over frequency range of 0.01 to 100 Hz, collecting 5 data points per decade, using a constant oscillatory stress of 0.5968 Pa and a temperature of 25°C. The tan delta is calculated as the ratio of G"/G'.

Record the G' and G" (Pa) at a frequency of 1 Hz.

### b) Ultracentrifuge Test

Compositions are considered to be in a single phase when they do not separate into layers or phases when ultracentrifuged for 2 hours at 50,000 rpm in an ultracentrifuge at 40°C (e.g., Beckman ultracentrifuge with swinging bucket rotor).

### c) Viscosity Method

To measure the viscosity of a personal care composition use a rheometer such as an AR G2 Rheometer (TA Instruments, DE, USA) equipped with 1 degree cone upper geometry with a diameter of 40 mm and flat plate lower geometry equipped with Peltier heating/cooling to control temperature. Measurement can be conducted by placing approximately 1 gram of personal care composition onto the lower test geometry and lowering the upper geometry into position to the desired gap of 52 microns, wiping away any excess composition to create an even surface around the edge of the geometry. Conduct a continuous flow test at 25°C, controlling the shear rate and progressing from a shear rate of 0.01 to 100 1/sec over a time period of 3 minutes, running the test in log mode and collecting 15 points per decade. Record the viscosity (PaS) at the shear rates of interest, for the samples herein we have reported the viscosity at a shear rate of 0.10 1/sec and 10 1/sec, interpolating as needed to obtain shear rates.

### d) Perfume Headspace Abundance During Dilution Method (PHADD)

### 1) Perfume headspace abundance for neat products

Unless otherwise indicated, all laboratory instruments are operated according to manufacturer's instructions. The following equipment is used: 20 mL headspace vials from Gerstel (Baltimore, MD); timer; gas chromatograph (GC) Agilent model 6890 and Gerstel MPS-2 auto sampler; GC column J&W DB5-MS, 30 m x 0.25 mm ID, 1.0 µm film thickness obtained from Agilent Technologies, Inc., Wilmington, DE, USA; carrier gas of ultra-pure helium, 1 mL/min. flow rate; solid-phase microextraction injection port liner (0.75mm ID) from Supelco; and a model 5973 Mass Selective Detector obtained from Agilent Technologies, Inc., Wilmington, DE, USA having a source temperature of 230 °C, and a MS Quad temperature of 150 °C.

1 gram of cleansing composition is placed into a clean 20mL headspace vial and a stir bar is added to the vial. A polytetrafluroethylene cap is placed on the vial and hand tightened. The sample is allowed to equilibrate to establish equilibrium of the perfume molecules between the composition and the headspace. This generally takes at least 30 minutes at room temperature. The sample is then analyzed using an automated solid phase microextraction-gas chromatograph-mass spectrometer (SPME-GC-MS) analysis system.

Transfer the sample vials to the auto sampler tray to begin analysis. Start the sequence of sample loading and analysis. Each sample vial is taken by the auto sampler to the incubation chamber and held at 30°C for 1 minute. The SPME fiber assembly is DVB/CAR/PDMS (50/30 um, 24 ga, 1 cm length). Sampling time is 1 minute. The samples are stirred at 500 rpm during SPME sampling. After sampling, the SPME fiber is injected into the GC injector. The injector temperature is 270°C.

The GC-MS analysis is started. SPME desorption time is 5 minutes. The following temperature program is used: i) an initial temperature of 50°C which is held for 0.5 minutes, and ii) increase the initial temperature at a rate of 8°C/min until a temperature of 275°C is reached, hold at 275°C for 2.5 minutes. Perfume compounds are identified using the MS spectral libraries of John Wiley & Sons and the National Institute of Standards and Technology (NIST), purchased and licensed through Agilent. Chromatographic peaks for specific ions are integrated using the MassHunter software obtained from Agilent Technologies, Inc., Wilmington, DE, USA. To calculate the perfume headspace abundance, all of the area counts of the perfume molecules are added together.

### 2) Perfume headspace abundance for diluted product

For the perfume headspace abundance for diluted product, use the method above for neat product, except 1.0 g of product is combined with sufficient water to reach the desired level of dilution, usually between (0.5-5g), a magnetic stir bar is added to each vial, and after the cap is in place, the vials are stirred via the magnetic bar for at least 30 minutes to equilibrate.

### e) Perfumed Skin Headspace Abundance Method (PSHAM)

Unless otherwise indicated, all laboratory instruments are operated according to manufacturer's instructions. The following equipment is used: Stir Bar Sorptive Extraction Sampling Devices - Gerstel Twister, 2 cm in length with 1 mm PDMS (polydimethyl silicone) phase thickness; glass sampling cups with magnets to hold Twisters during sampling - 35 mL in volume; timer; gas chromatograph (GC) Agilent model 7890 and Gerstel MPS-2 autosampler with thermal desorption unit (TDU) and cooled-on-column (CIS-4) temperature programmable inlet; GC column J&W DB5-MS, 30 m x 0.25 mm ID, 1.00 µm film thickness obtained from Agilent Technologies, Inc., Wilmington, DE, USA; carrier gas of ultra-pure helium, 1 mL/min. flow rate; liquid nitrogen for injection port cryogenic cooling; Gerstel TDU injection port liners with glass wool; and a detector model 5975 Mass Selective Detector obtained from Agilent Technologies, Inc., Wilmington, DE, USA having a source temperature of 230°C, and a MS Quad temperature of 150°C.

Headspace samples are collected from panelists' arms that have been washed with test products and/or controls. The wash protocol includes: 1) adjusting water temperature to 100°F and water flow to 1 gallon/min; 2) rinsing the arm under the water stream for 5 seconds; 3) apply product of known weight on a puff which has been prewetted for 5 seconds with water; 4) lather product in the puff by hands for 10 seconds; 5) wash the entire forearm for 15 seconds using back and forth motion, then wait for 15 seconds; 6) rinse the arm under the water stream for 15 seconds; 7) pat dry the forearm using a paper towel; and then 8) proceed to sensory evaluation or analytical sampling. The Twister device is held inside of the sampling cup with magnetic force while the cup is placed against panelists' arms for a period of 3 minutes. The Twister is then transferred to the thermal desorption tube and capped with a transport adapter.

To begin the analysis, transfer the Twister transport tubes to the autosampler tray and proceed with TDU-GC-MS analysis. Set-up the sequence of samples needing to be analyzed and start the sequence of sample loading and analysis. In this step, the Twister transport tube is taken by the autosampler to the thermal desorption unit where it is heated to 250°C and held at that temperature for 5 minutes. Perfume materials that are thermally desorbed from the Twister are trapped by the liquid nitrogen cooled inlet, which is held at -120°C during desorption. The programmable temperature inlet is then heated 275°C and held at that temperature for 3 minutes.

The GC-MS analysis run is started and the GC temperature program is initiated with mass spectrometer detection. The following temperature program is used: i) an initial temperature of 50°C which is held for 0.5 minutes, and ii) increase the initial temperature at a rate of 8°C/min until a temperature of 275°C is reached, hold at 275°C for 5 minutes. Perfume compounds are identified using the MS spectral libraries of John Wiley & Sons and the National Institute of Standards and Technology (NIST), purchased and licensed through Agilent. Chromatographic peaks for specific ions are integrated using the MassHunter software obtained from Agilent Technologies, Inc., Wilmington, DE, USA. Abundance of perfume in the headspace over the skin is calculated by adding the area counts of all the perfume molecules. The relative enhancement of abundance of perfume in the headspace using test products over control products is obtained by the ratio of the total peak area counts. The PSHAM measurement may be repeated on the target surface at later time intervals to test for longevity of fragrance on the skin. These time intervals could be any desired, for example, 1 hour, 2 hours, 3 hours, 3.5 hours, 4 hours, etc. after the initial application.

### f) Cylinder Method

Lather can be measured in accordance with the Cylinder Method. Lather volume is measured using a graduated cylinder and a rotating mechanical apparatus. A 1,000 ml graduated cylinder is used which is marked in 10 ml increments, has a height of 14.5 inches at the 1,000 ml mark from the inside of its base, and has a neck at its top fitted for a plastic insert cap (for example, Pyrex No. 2982). Moderately hard water is prepared with 1.5:1 ion ratio Ca/Mg by dissolving 1.14 grams calcium chloride dihydrate and 1.73 grams magnesium chloride hexahydrate into one U.S. gallon distilled water. The water is maintained at between 105 - 110 °F. The graduated cylinder is heated to the same temperature by flushing with excess tap water at the same temperature for 15 seconds, then drying it outside and shaking briefly upside down to dry the interior. 100.0 grams of the moderately hard water at the indicated temperature is weighed directly into the graduated cylinder. The cylinder is clamped in a mechanical rotating device, which clamps the cylinder vertically with an axis of rotation that transects the center of the graduated cylinder. Using a 3- or 4-place metric balance, invert the plastic cap for the graduated cylinder onto the balance pan and weigh 0.500 grams of composition (for compositions less than 19% surfactant) to within 4 milligrams accuracy, using a holder to keep the cap level. When the surfactant level is 40% or greater, use 125 mg of composition (500 g/4). When it is between 30% and 39%, use 135 mg of composition, and when it is between 20% and 29% use 250 mg and for 19 wt% and below use 500 mg. Insert the cap into the graduated cylinder neck while being careful that all composition is now in the space in the cylinder interior. For compositions with very low viscosity which will not remain on the cap surface, 500 mg composition can be added directly to the graduated cylinder. Rotate the cylinder for 25 complete revolutions at a rate of 10 revolutions per 18 seconds to create a lather and stop in a level, vertical position. When the cylinder stops in a vertical position, start a digital stopwatch. Observing the water draining at the bottom, record the time to the nearest second when the water height measures 50 cc, then 60 cc, then 70 cc and so on until at least 90 cc has drained. Measure and record the total height of the foam in the column interior, which is the lather volume. If the top surface of the lather is uneven, the lowest height at which it is possible to see halfway across the graduated cylinder is the lather volume (ml). If the lather is coarse such that a single or only a few foam cells ("bubbles") reach across the entire cylinder, the height at which at least 10 foam cells are required to fill the space is the lather volume, also in ml up from the base. When measuring the lather height, bubbles that are larger than 1 inch across at the top surface are considered free air and not lather. The measurement is repeated and at least three results averaged to obtain the lather volume. In a spreadsheet, calculate the lather density at each observed time point as the volume of foam (total height minus water height) divided by the weight of the foam (100.5 grams minus the weight of water observed, using a density of 1.00 g/cc for water). Fit the 3 time points closest to (ideally, also bracketing) 20 seconds to a 2^{nd} order polynomial equation. Solve the equation for the lather density at 20 seconds, which is the lather density of the composition. Multiply the lather volume by the lather density to obtain the lather mass, in grams.

The entire process should take less than 3 minutes in order to maintain desired temperature.

### g) Stability Test

A composition is filled into a 4 fl. oz. glass jar with minimal headspace and capped, placed in a dark room maintained at 40°C for 3 months. A composition is stable if there is minimal visual sign of phase separation and the viscosity changes by less than 90% from the original viscosity.

## Claims

1. Use of a hydric solvent to enhance fragrance longevity on skin of a rinse-off cleansing composition, wherein the composition comprises: a) from 35% to 85%, by weight of the composition, of surfactant, wherein the surfactant comprises a first surfactant and a cosurfactant; b) from 4% to 30%, by weight of the composition, of a perfume, wherein the weight percent of perfume is from 8% to 90%, by weight of the surfactant; c) from 6% to 20%, by weight of the composition, of a hydric solvent, wherein the hydric solvent comprises dipropylene glycol, diethylene glycol, dibutylene glycol, hexylene glycol, butylene glycol, pentanediol, heptanediol, propylene glycol, a polyethylene glycol having an average molecular weight below 500, or a combination thereof, and wherein the weight percent of the hydric solvent is from 16% to 24%, by weight of the surfactant; and d) from 2% to 57%, by weight of the composition, of water; wherein the rinse-off cleansing composition is not a ringing gel, and wherein the composition is a microemulsion or contains a microemulsion phase.

2. The use of claim 1, wherein the composition has an elastic modulus G' at 1 Hz of 25 Pa to 3000 Pa, preferably 50 Pa to 2500 Pa, more preferably from 100 Pa to 1500 Pa, or even more preferably from 150 Pa to 1000 Pa, as measured according to the G' and G" Test Method as disclosed herein.

3. The use of any preceding claim, wherein the composition has a total GCMS count higher than that of a control where the solvent is replaced with water when the total GCMS count is measured in accordance with the PSHAM method at 1 hour after the initial application, preferably is at least about 10% more than the control, 15%, 20%, 25%, 50%, 75%, 100%, 150%, 200%, 250%, in increasing order of preference, and most preferably at least 300%.

4. The use of any preceding claim, wherein the composition comprises from 35% to 80%, preferably from 35% to 70%, more preferably from 35% to 60%, and even more preferably from 35% to 45%, by weight of the composition, of surfactant.

5. The use of any preceding claim, wherein the first surfactant comprises a branched anionic surfactant.

6. The use of claim 5, wherein the first surfactant comprises sodium trideceth-2 sulfate, sodium trideceth-3 sulfate, or a combination thereof.

7. The use of any preceding claim, wherein the cosurfactant comprises a betaine, preferably an alkyl amidopropyl betaine, or more preferably cocamidopropyl betaine.

8. The use of any preceding claim, wherein the composition comprises from 6% to 30%, preferably from 7% to 25%, more preferably from 8% to 20%, or even more preferably from 8% to 15%, by weight of the composition, of the perfume.

9. The use of any preceding claim, wherein the composition has from 7% to 18%, preferably from 8% to 16%, more preferably from 9% to 15%, or even more preferably from 10% to 14%, by weight of the composition, of the hydric solvent.

10. The use of any preceding claim, wherein the hydric solvent comprises dipropylene glycol.

11. The use of any preceding claim, wherein the composition comprises from 8% to 75%, preferably from 15% to 70%, more preferably from 25% to 65%, or even more preferably from 30% to 61%, by weight of the composition, of the combination of water and hydric solvent.

12. The use of any preceding claim, wherein at least a portion of the composition becomes a microemulsion upon dilution with water of 3:1 by weight (water:composition) to 10:1 by weight (water:composition).

13. A method of enhancing fragrance longevity on skin of a rinse-off cleansing composition, comprising, combining a) from 35% to 85%, by weight of the composition, of surfactant; b) from 4% to 30%, by weight of the composition, of a perfume, wherein the weight percent of perfume is from 12% to 40%, by weight of the surfactant; c) from 6% to 20%, by weight of the composition, of a hydric solvent, wherein the hydric solvent comprises dipropylene glycol, diethylene glycol, dibutylene glycol, hexylene glycol, butylene glycol, pentanediol, heptanediol, propylene glycol, a polyethylene glycol having an average molecular weight below 500, or a combination thereof, preferably dipropylene glycol, and wherein the weight percent of the hydric solvent is from 16% to 24%, by weight of the surfactant; and d) from 2% to 57%, by weight of the composition, of water; to form the cleansing composition; wherein the rinse-off cleansing composition is not a ringing gel, and wherein the composition is a microemulsion or contains a microemulsion phase.

## Patentansprüche

1. Verwendung eines wasserhaltigen Lösungsmittels zum Verbessern der Duftstofflebensdauer einer Spülreinigungszusammensetzung auf der Haut, wobei die Zusammensetzung Folgendes umfasst: a) zu 35 Gew-% bis 85 Gew.-% der Zusammensetzung Tensid, wobei das Tensid ein erstes Tensid und ein Cotensid umfasst; b) zu 4 Gew-% bis 30 Gew.-% der Zusammensetzung einen Duftstoff, wobei der Gewichtsprozentsatz des Duftstoffes 8 Gew-% bis 90 Gew.-% des Tensids beträgt; c) zu 6 Gew-% bis 20 Gew.-% der Zusammensetzung ein wasserhaltiges Lösungsmittel, wobei das wasserhaltige Lösungsmittel Dipropylenglykol, Diethylenglykol, Dibutylenglykol, Hexylenglykol, Butylenglykol, Pentandiol, Heptandiol, Propylenglykol, ein Polyethylenglykol mit einem durchschnittlichen Molekulargewicht von unter 500, oder eine Kombination davon umfasst, und wobei der Gewichtsprozentsatz des wasserhaltigen Lösungsmittels 16 Gew-% bis 24 Gew.-% des Tensids beträgt; und d) zu 2 Gew.-% bis 57 Gew.-% der Zusammensetzung Wasser; wobei die Spülreinigungszusammensetzung kein Brummgel ist, und wobei die Zusammensetzung eine Mikroemulsion ist oder eine Mikroemulsionsphase enthält.

2. Verwendung nach Anspruch 1, wobei die Zusammensetzung bei 1 Hz einen Elastizitätsmodul G' von 25 Pa bis 3000 Pa aufweist, vorzugsweise 50 Pa bis 2500 Pa, mehr bevorzugt von 100 Pa bis 1500 Pa, oder noch mehr bevorzugt von 150 Pa bis 1000 Pa, gemessen gemäß dem hier offenbarten G'- und G"-Prüfverfahren.

3. Verwendung nach einem der vorstehenden Ansprüche, wobei die Zusammensetzung eine GCMS-Gesamtzahl aufweist, die höher ist als die einer Kontrolle, wo das Lösungsmittel durch Wasser ersetzt ist, wenn die GCMS-Gesamtzahl gemäß dem PSHAM-Verfahren 1 Stunde nach der ersten Anwendung gemessen wird, vorzugsweise mindestens etwa 10 % höher liegt als die Kontrolle, 15 %, 20 %, 25 %, 50 %, 75 %, 100 %, 150 %, 200 %, 250 %, in aufsteigender Reihenfolge der Präferenz, und am meisten bevorzugt mindestens 300 %.

4. Verwendung nach einem der vorstehenden Ansprüche, wobei die Zusammensetzung zu 35 Gew.-% bis 80 Gew.-%, vorzugsweise zu 35 Gew.-% bis 70 Gew.-%, mehr bevorzugt zu 35 Gew.-% bis 60 Gew.-%, und noch mehr bevorzugt zu 35 Gew.-% bis 45 Gew.-% der Zusammensetzung Tensid umfasst.

5. Verwendung nach einem der vorstehenden Ansprüche, wobei das erste Tensid ein verzweigtes anionisches Tensid umfasst.

6. Verwendung nach Anspruch 5, wobei das erste Tensid Natriumtrideceth-2-sulfat, Natriumtrideceth-3-sulfat oder eine Kombination davon umfasst.

7. Verwendung nach einem der vorstehenden Ansprüche, wobei das Cotensid ein Betain, vorzugsweise ein Alkylamidopropylbetain oder mehr bevorzugt Cocamidopropylbetain umfasst.

8. Verwendung nach einem der vorstehenden Ansprüche, wobei die Zusammensetzung zu 6 Gew.-% bis 30 Gew.-%, vorzugsweise zu 7 Gew.-% bis 25 Gew.-%, mehr bevorzugt zu 8 Gew.-% bis 20 Gew.-%, oder noch mehr bevorzugt zu 8 Gew.-% bis 15 Gew.-% der Zusammensetzung den Duftstoff umfasst.

9. Verwendung nach einem der vorstehenden Ansprüche, wobei die Zusammensetzung zu 7 Gew.-% bis 18 Gew.-%, vorzugsweise zu 8 Gew.-% bis 16 Gew.-%, mehr bevorzugt zu 9 Gew.-% bis 15 Gew.-%, oder noch mehr bevorzugt zu 10 Gew.-% bis 14 Gew.-% der Zusammensetzung das wasserhaltige Lösungsmittel aufweist.

10. Verwendung nach einem der vorstehenden Ansprüche, wobei das wasserhaltige Lösungsmittel Dipropylenglykol umfasst.

11. Verwendung nach einem der vorstehenden Ansprüche, wobei die Zusammensetzung zu 8 Gew.-% bis 75 Gew.-%, vorzugsweise zu 15 Gew-% bis 70 Gew.-%, mehr bevorzugt zu 25 Gew.-% bis 65 Gew.-%, oder noch mehr bevorzugt zu 30 Gew.-% bis 61 Gew.-% der Zusammensetzung die Kombination von Wasser und wasserhaltigem Lösungsmittel umfasst.

12. Verwendung nach einem der vorstehenden Ansprüche, wobei zumindest ein Teil der Zusammensetzung bei Verdünnung mit Wasser von 3:1, bezogen auf das Gewicht (Wasser:Zusammensetzung), bis 10:1, bezogen auf das Gewicht (Wasser:Zusammensetzung), eine Mikroemulsion wird.

13. Verfahren zum Verbessern der Duftstofflebensdauer einer Spülreinigungszusammensetzung auf der Haut, umfassend Kombinieren a) zu 35 Gew.-% bis 85 Gew.-% der Zusammensetzung Tensid; b) zu 4 Gew.-% bis 30 Gew.-% der Zusammensetzung einen Duftstoff, wobei der Gewichtsprozentsatz des Duftstoffes 12 Gew.-% bis 40 Gew.-% des Tensids beträgt; c) zu 6 Gew.-% bis 20 Gew.-% der Zusammensetzung ein wasserhaltiges Lösungsmittel, wobei das wasserhaltige Lösungsmittel Dipropylenglykol, Diethylenglykol, Dibutylenglykol, Hexylenglykol, Butylenglykol, Pentandiol, Heptandiol, Propylenglykol, ein Polyethylenglykol mit einem mittleren Molekulargewicht von unter 500 oder eine Kombination davon umfasst, vorzugsweise Dipropylenglykol, und wobei der Gewichtsprozentsatz des wasserhaltigen Lösungsmittels 16 Gew.-% bis 24 Gew.-% des Tensids beträgt; und d) zu 2 Gew.-% bis 57 Gew.-% der Zusammensetzung Wasser; zur Bildung der Reinigungszusammensetzung; wobei die Spülreinigungszusammensetzung kein Brummgel ist, und wobei die Zusammensetzung eine Mikroemulsion ist oder eine Mikroemulsionsphase enthält.

## Revendications

1. Utilisation d'un solvant hydrique pour améliorer la longévité de parfum sur la peau d'une composition de nettoyage à éliminer par rinçage, dans laquelle la composition comprend : a) de 35 % à 85 %, en poids de la composition, d'agent tensioactif, dans laquelle l'agent tensioactif comprend un premier agent tensioactif et un co-tensioactif ; b) de 4 % à 30 %, en poids de la composition, d'un parfum, dans lequel le pourcentage en poids de parfum va de 8 % à 90 %, en poids de l'agent tensioactif ; c) de 6 % à 20 %, en poids de la composition, d'un solvant hydrique, dans laquelle le solvant hydrique comprend du dipropylène glycol, du diéthylène glycol, du dibutylène glycol, de l'hexylène glycol, du butylène glycol, du pentane-diol, de l'heptane-diol, du propylène glycol, un polyéthylène glycol ayant une masse moléculaire moyenne inférieure à 500, ou une combinaison de ceux-ci, et dans laquelle le pourcentage en poids du solvant hydrique va de 16 % à 24 %, en poids de l'agent tensioactif ; et d) de 2 % à 57 %, en poids de la composition, d'eau ; dans laquelle la composition de nettoyage à éliminer par rinçage n'est pas un gel vibrant, et dans laquelle la composition est une micro-émulsion ou contient une phase micro-émulsion.

2. Utilisation selon la revendication 1, dans laquelle la composition a un module élastique G' à 1 Hz de 25 Pa à 3000 Pa, de préférence 50 Pa à 2500 Pa, plus préférablement de 100 Pa à 1500 Pa, ou même plus préférablement de 150 Pa à 1000 Pa, tel que mesuré selon le procédé de test de G' et G" tel que décrit ici.

3. Utilisation selon une quelconque revendication précédente, dans laquelle la composition a un comptage total GCMS supérieur à celui d'un témoin où le solvant est remplacé par de l'eau lorsque le comptage total GCMS est mesuré conformément au procédé PSHAM 1 heure après l'application initiale, de préférence est au moins environ 10 % supérieur au témoin, 15 %, 20 %, 25 %, 50 %, 75 %, 100 %, 150 %, 200 %, 250 %, dans un ordre croissant de préférence, et le plus préférablement au moins 300 %.

4. Utilisation selon une quelconque revendication précédente, dans laquelle la composition comprend de 35 % à 80 %, de préférence de 35 % à 70 %, plus préférablement de 35 % à 60 %, et même plus préférablement de 35 % à 45 %, en poids de la composition, d'agent tensioactif.

5. Utilisation selon une quelconque revendication précédente, dans laquelle le premier agent tensioactif comprend un agent tensioactif anionique ramifié.

6. Utilisation selon la revendication 5, dans laquelle le premier agent tensioactif comprend du tridéceth-2 sulfate de sodium, du tridéceth-3 sulfate de sodium, ou une combinaison de ceux-ci.

7. Utilisation selon une quelconque revendication précédente, dans laquelle le co-tensioactif comprend une bétaïne, de préférence une alkyl-amidopropyl-bétaïne, ou plus préférablement de la cocamidopropyl-bétaïne.

8. Utilisation selon une quelconque revendication précédente, dans laquelle la composition comprend de 6 % à 30 %, de préférence de 7 % à 25 %, plus préférablement de 8 % à 20 %, ou même plus préférablement de 8 % à 15 %, en poids de la composition, du parfum.

9. Utilisation selon une quelconque revendication précédente, dans laquelle la composition a de 7 % à 18 %, de préférence de 8 % à 16 %, plus préférablement de 9 % à 15 %, ou même plus préférablement de 10 % à 14 %, en poids de la composition, du solvant hydrique.

10. Utilisation selon une quelconque revendication précédente, dans laquelle le solvant hydrique comprend du dipropylène glycol.

11. Utilisation selon une quelconque revendication précédente, dans laquelle la composition comprend de 8 % à 75 %, de préférence de 15 % à 70 %, plus préférablement de 25 % à 65 %, ou même plus préférablement de 30 % à 61 %, en poids de la composition, de la combinaison d'eau et de solvant hydrique.

12. Utilisation selon une quelconque revendication précédente, dans laquelle au moins une partie de la composition devient une micro-émulsion lors d'une dilution avec de l'eau de 3:1 en poids (eau:composition) à 10:1 en poids (eau:composition).

13. Procédé d'amélioration de la longévité de parfum sur la peau d'une composition de nettoyage à éliminer par rinçage, comprenant, la combinaison a) de 35 % à 85 %, en poids de la composition, d'agent tensioactif ; b) de 4 % à 30 %, en poids de la composition, d'un parfum, dans lequel le pourcentage en poids de parfum va de 12 % à 40 %, en poids de l'agent tensioactif ; c) de 6 % à 20 %, en poids de la composition, d'un solvant hydrique, dans laquelle le solvant hydrique comprend du dipropylène glycol, du diéthylène glycol, du dibutylène glycol, de l'hexylène glycol, du butylène glycol, du pentane-diol, de l'heptane-diol, du propylène glycol, un polyéthylène glycol ayant une masse moléculaire moyenne inférieure à 500, ou une combinaison de ceux-ci, de préférence du dipropylène glycol, et dans laquelle le pourcentage en poids du solvant hydrique va de 16 % à 24 %, en poids de l'agent tensioactif ; et d) de 2 % à 57 %, en poids de la composition, d'eau ; pour former la composition de nettoyage ; dans laquelle la composition de nettoyage à éliminer par rinçage n'est pas un gel vibrant, et dans laquelle la composition est une micro-émulsion ou contient une phase micro-émulsion.
